# EUROPÄISCHE PATENTSCHRIFT

(11) **EP 1 496 876 B1**
(45) Veröffentlichungstag und Bekanntmachung des Hinweises auf die Patenterteilung: **19.03.2008**
(21) Anmeldenummer: 03718724.2
(22) Anmeldetag: 02.04.2003
(51) Int. Cl.: A61K 9/72, A61K 31/46, A61K 31/5386

(54) **AEROSOLFORMULIERUNG FÜR DIE INHALATION ENTHALTEND TIOTROPIUMBROMID**
AEROSOL FORMULATION FOR INHALATION COMPRISING TIOTROPIUMBROMIDE
FORMULATION D'AEROSOL CONTENANT TIOTROPIUM BROMIDE POUR ADMINISTRATION PAR INHALATION

(30) Priorität: 11.04.2002 DE 10216036
(43) Veröffentlichungstag der Anmeldung: 19.01.2005
(73) Patentinhaber: Boehringer Ingelheim Pharma GmbH & Co.KG, 55216 Ingelheim am Rhein (DE)
(72) Erfinder: DRECHSEL, Karin, 68161 Mannheim (DE); NIKLAUS-HUMKE, Barbara, 55432 Damscheid (DE); SCHMELZER, Christel, 55218 Ingelheim (DE); BARTH, Petra, 55129 Mainz (DE)
(86) Internationale Anmeldenummer: PCT/EP2003/003438
(87) Internationale Veröffentlichungsnummer: WO 2003/084519

(56) Entgegenhaltungen:
- WO-A-00/23037
- WO-A-02/36104
- WO-A-98/27959

## Beschreibung

Die vorliegend Erfindung betrifft eine treibgasfreie Aerosolformulierung von in Wasser gelöstem Tiotropiumbromid. Die erfindungsgemäße Formulierung eignet sich besonders zum Vernebeln des Wirkstoffs mittels eines Zerstäubers, um den Wirkstoff inhalativ zu applizieren. Bevorzugte Indikationen sind Asthma und / oder COPD.

Tiotropium, chemisch (1α,2β,4β,5α,7β)-7-[(hydroxydi-2-thienylacetyl)oxy]-9,9-dimethyl-3-oxa-9-azoniatricyclo[3.3.1.0]nonane, ist als Tiotropiumbromid aus der Europäischen Patentanmeldung EP 418 716 A1 bekannt. Das Bromid-Salz des Tiotropiums weist die folgende chemische Struktur auf:

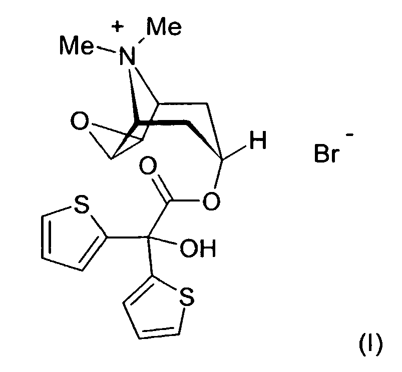

Die Verbindung besitzt wertvolle pharmakologische Eigenschaften und ist unter dem Namen Tiotropiumbromid bekannt. Tiotropium und seine Salze stellen ein hochwirksame Anticholinergika dar und können deshalb bei der Therapie von Asthma oder COPD (chronic obstructive pulmonary disease = chronisch obstruktive Lungenerkrankung) einen therapeutischen Nutzen entfalten. Pharmakologisch ebenfalls interessant ist das Monohydrat des Tiotropiumbromids.

Beide Verbindungen sind bevorzugter Gegenstand der vorliegenden Erfindung.

Die vorliegende Erfindung beschäftigt sich mit inhalativ applizierbaren flüssigen Wirkstoffformulierungen dieser Verbindungen, wobei die erfindungsgemäßen flüssigen Formulierungen hohen Qualitätsstandards genügen müssen.

Um eine optimale Wirkstoffverteilung der Wirksubstanzen in der Lunge zu erhalten, bietet sich die Applikation einer flüssigen, auf Treibgase verzichtenden, Formulierung mittels dafür geeignet Inhalatoren an. Besonders geeignet sind solche Inhalatoren, die eine kleine Menge einer flüssigen Formulierung in der therapeutisch notwendigen Dosierung binnen weniger Sekunden in ein therapeutisch-inhalativ geeignetes Aerosol vernebeln können. Im Rahmen der vorliegenden Erfindung sind solche Vernebler bevorzugt, bei denen bereits eine Menge von weniger als 100 Mikroliter, bevorzugt weniger als 50 Mikroliter, ganz bevorzugt weniger als 20 Mikroliter Wirkstofflösung mit bevorzugt einem Hub oder zwei Hüben zu einem Aerosol mit einer durchschnittlichen Teilchengröße von weniger als 20 Mikrometern, bevorzugt weniger als 10 Mikrometern, so vernebelt werden können, daß der inhalierbare Anteil des Aerosols bereits der therapeutisch wirksamen Menge entspricht.

Eine derartige Vorrichtung zur treibgasfreien Verabreichung einer dosierten Menge eines flüssigen Arzneimittels zur inhalativen Anwendung, wird beispielsweise in der internationalen Patentanmeldung WO 91/14468 "Atomizing Device and Methods" als auch in der WO 97/12687, dort Figuren 6a und 6b und der dazugehörigen Beschreibung, ausführlich beschrieben. In einem solchen Vernebler wird eine Arzneimittellösung mittels hohen Drucks von bis zu 500 bar in ein lungengängiges Aerosol überführt und versprüht. Auf die genannten Referenzen wird im Rahmen der vorliegenden Erfindungsbeschreibung ausdrücklich in Gänze Bezug genommen.

In solchen Inhalatoren werden die Lösungsformulierungen in einem Reservoir gelagert. Dabei ist es notwendig, daß die verwendeten Wirkstoffformulierungen eine ausreichende Lagerstabilität aufweisen und gleichzeitig so beschaffen sind, daß sie dem medizinischen Zweck entsprechend möglichst ohne weitere Manipulation, direkt appliziert werden können. Ferner dürfen sie keine Bestandteile aufweisen, die so mit dem Inhalator wechselwirken können, daß der Inhalator oder die pharmazeutische Qualität der Lösung, respektive des erzeugten Aerosols, Schaden nehmen könnte.

Zur Vernebelung der Lösung wird eine spezielle Düse verwendet, wie sie beispielsweise die WO 94/07607 oder die WO 99/16530 beschreiben.

Die WO 98/27959 offenbart Lösungsformulierungen für den oben beschriebenen Inhalator, die als Zusatz das Dinatriumsalz der Editinsäure (bzw. Dinatriumethylendiamintetraacetat-Dihydrat oder Natriumedetat) enthalten. Die Schrift favorisiert für wässrige Lösungsformulierungen, die mit Hilfe des eingangs beschriebenen Inhalators in inhalierbare Aerosole versprüht werden sollen, eine Mindestkonzentration an Natriumedetat von 50 mg/100 ml um die Inzidenz von Sprühanomalien zu verringern. Unter den offenbarten Beispielen findet sich eine Formulierung mit Tiotropiumbromid mit einem pH-Wert von 3,2 bzw. 3,4. Bei dieser Formulierung ist der Wirkstoff in Wasser gelöst. Der Anteil an Natriumedetat beträgt ebenfalls 50 mg / 100 ml.

WO 00/23037 offenbart ein Wirkstoffkonzentrat mit Tiotropiumbromid mit einem pH-Wert von 3.0. Die Mindestkonzentration an Natriumedetat Geträgt 50 mg/100 ml.

Überraschend wurde jetzt allerdings gefunden, daß wässrige Lösungsformulierungen von Tiotropiumsalzen dann besonders stabil sind, wenn der pH-Wert unter 3,2, bevorzugt unter 3,1 liegt.

Darüber hinaus wurde gefunden, dass derartige Formulierungen gegenüber der aus dem Stand der Technik bekannten Formulierung mit Tiotropiumbromid bei Vernebelung mittels des Inhalators Respimat® eine Reduktion der Streuung der ausgebrachten Masse aufweist, wenn die Menge an Natriumedetat zwischen 5 mg und 20 mg pro 100 g Formulierung liegt. Die Sprayqualität der erfindungsgemäßen Formulierung ist sehr gut. Ein so generiertes Aerosol weist für die inhalative Applikation sehr gute Eigenschaften auf.

Außerdem weist die erfindungsgemäße Formulierung eine verbesserte Stabilität auf und vermindert die Belastung des Patienten mit Natriumedetat.

Es ist daher eine Aufgabe der vorliegenden Erfindung, eine wässrige Wirkstoffformulierung mit einem pharmazeutisch akzeptablen Tiotropiumsalz zu schaffen, welche den hohen Standards genügt, die notwendig sind, um eine Lösung mittels der eingangs genannten Inhalatoren optimal vernebeln zu können. Die erfindungsgemäßen Wirkstoffformulierungen müssen dabei auch eine ausreichende pharmazeutische Qualität aufweisen, d.h. sie sollten über eine Lagerzeit von einigen Jahren, bevorzugt von mindestens einem Jahr, stärker bevorzugt von zwei Jahren pharmazeutisch stabil sein.

Eine weitere Aufgabe besteht darin, treibgasfreie Lösungsformulierungen mit Tiotropiumsalzen zu schaffen, die mittels eines Inhalators unter Druck vernebelt werden, wobei die im generierten Aerosol ausgebrachte Masse reproduzierbar innerhalb eines definierten Bereichs liegt.

Eine weitere Aufgabe besteht darin, eine inhalierbare Formulierung mit Tiotropiumbromid als flüssige Formulierung mit Wasser als Lösungsmittel zu schaffen, welche stabil ist und die Belastung des Patienten mit chemischen Stoffen auf ein Minimum reduziert.

Im Rahmen der vorliegenden Erfindung ist als Salz das Tiotropiumbromid bevorzugt. Bezugnahmen auf Tiotropiumbromid sind im Rahmen der vorliegenden Erfindung stets als Bezugnahmen auf alle möglichen amorphen und kristallinen Modifikationen des Tiotropiumbromids zu verstehen. Diese können beispielsweise in der kristallinen Struktur Lösemittelmoleküle mit einschließen. Von allen kristallinen Modifikationen des Tiotropiumbromids sind erfindungsgemäß diejenigen, die Wasser mit einschließen (Hydrate) bevorzugt. Besonders bevorzugt ist im Rahmen der vorliegenden Erfindung das Tiotropiumbromid-Monohydrat einsetzbar. Die Formulierung enthält bevorzugt keinen weiteren Wirkstoff, der nicht Tiotropium enthält.

In der erfindungsgemäßen Formulierung liegt das Tiotropiumbromid in Wasser gelöst vor. Ein weiteres Lösungsmittel wird nicht verwendet. Die Formulierung ist insbesondere frei von Treibgasen.

Erfindungsgemäß enthält die Formulierung Tiotropiumbromid oder Tiotropiumbromid-Monohydrat.

Allerdings kann die Formulierung auch ein Gemisch verschiedener Tiotropiumsalze und Solvate enthalten.

Die Konzentration des Tiotropiumsalzes bezogen auf den Anteil an Tiotropium in der fertigen Arzneimittelzubereitung ist abhängig von dem angestrebten therapeutischen Effekt. Für die Mehrzahl der auf Tiotropium ansprechenden Erkrankungen liegt die Konzentration an Tiotropium zwischen 0,01 g pro 100 g Formulierung und 0,06 g pro 100 g Formulierung. Da die Dichte der Formulierung 1,00 g/cm³ liegt, entsprechen die 100 g Formulierung einem Volumen von 100 ml. Im Rahmen der vorliegenden Erfindungsbeschreibung bedeutet die Bezeichnung pro 100 ml bzw. / 100 ml jeweils pro 100 ml Formulierung, sofern nicht anders gekennzeichnet. Bevorzugt ist eine Menge von 0,015 g /100 ml bis 0,055 g / 100 ml, stärker bevorzugt ist eine Menge von 0,02 g / 100 ml bis 0,05 g / 100 ml. Am stärksten bevorzugt ist eine Menge von 0,023 ± 0,001g pro 100 ml Formulierung bis zu 0,045 ± 0,001g pro 100 ml Formulierung.

Der pH-Wert der erfindungsgemäßen Formulierung liegt zwischen 2,7 und 3,1, bevorzugt zwischen 2,8 und 3,05, stärker bevorzugt zwischen 2,80 und 3,0 und noch stärker bevorzugt bei 2,9.

Der pH-Wert wird durch Zugabe von pharmakologisch verträglichen Säuren eingestellt.

Beispiele für diesbezüglich bevorzugte anorganische Säuren sind: Salzsäure, Bromwasserstoffsäure, Salpetersäure, Schwefelsäure und/oder Phosphorsäure. Beispiele für besonders geeignete organische Säuren sind: Ascorbinsäure, Zitronensäure, Äpfelsäure, Weinsäure, Maleinsäure, Bernsteinsäure, Fumarsäure, Essigsäure, Ameisensäure und/oder Propionsäure und andere. Bevorzugte anorganische Säuren sind Salzsäure, Schwefelsäure. Es können auch die Säuren verwendet werden, die bereits mit dem Wirkstoff ein Säureadditionssalz bilden.

Unter den organischen Säuren sind Ascorbinsäure, Fumarsäure und Zitronensäure bevorzugt, wobei Zitronensäure am stärksten bevorzugt ist. Gegebenenfalls können auch Gemische der genannten Säuren eingesetzt werden, insbesondere in Fällen von Säuren, die neben ihren Säuerungseigenschaften auch andere Eigenschaften, z.B. als Geschmackstoffe oder Antioxidantien besitzen, wie beispielsweise Zitronensäure oder Ascorbinsäure. Unter den genannten Säuren werden ausdrücklich Salzsäure und Zitronensäure als am Stärksten bevorzugt genannt.

Gegebenenfalls können auch pharmakologisch verträgliche Basen zum genauen Austitrieren des pH-Wertes eingesetzt werden. Als Basen eignen sich beispielsweise Alkalihydroxide und Alkalicarbonate. Bevorzugtes Alkaliion ist Natrium. Werden solche Basen verwendet, ist darauf zu achten, daß auch die daraus resultierenden Salze, die dann in der fertigen Arzneimittelformulierung enthalten sind, mit der oben genannten Säure pharmakologisch akzeptabel sind.

Erfindungsgemäß enthält die Formulierung Editinsäure (EDTA) oder ein bekanntes Salze davon, z.B. Natrium-EDTA, bzw. Dinatrium-EDTA-Dihydrat (Natriumedetat) als Stabilisator oder Komplexbildner. Bevorzugt wird Natriumedetat eingesetzt.

Der Gehalt bezogen auf Natriumedetat liegt dabei zwischen 5 mg / 100 ml Formulierung und 20 mg / 100 ml Formulierung, bevorzugt zwischen 5 mg / 100 ml Formulierung und 15 mg / 100 ml Formulierung, stärker bevorzugt zwischen 8 mg / 100 ml Formulierung und 12 mg / 100 ml Formulierung, am stärksten bevorzugt bei 10 mg / 100 ml Formulierung.

Wird ein anders Salz der Editinsäure verwendet oder die Säure als solche werden analoge Mengen des Komplexbildners eingesetzt.

Analoges wie bereits für Natriumedetat ausgeführt, gilt auch für mögliche, wenn auch gegenüber EDTA oder seinen Salzen nicht bevorzugte andere vergleichbare Zusatzstoffe, die komplexbildende Eigenschaften aufweisen und anstelle dessen verwendet werden können, wie beispielsweise Nitrilotriessigsäure und deren Salze.

Unter Komplexbildner werden im Rahmen der vorliegenden Erfindung bevorzugt Moleküle verstanden, die in der Lage sind Komplexbindungen einzugehen. Bevorzugt sollen durch diese Verbindungen Kationen, besonders bevorzugt metallische Kationen komplexiert werden.

Der erfindungsgemäßen Formulierung können weitere pharmakologisch verträgliche Hilfsstoffe zugesetzt werden.

Unter Hilfs- und Zusatzstoffen wird in diesem Zusammenhang jeder pharmakologisch verträgliche und therapeutisch sinnvolle Stoff verstanden, der kein Wirkstoff ist, aber zusammen mit dem Wirkstoff in dem pharmakologisch geeigneten Lösungsmittel formuliert werden kann, um die qualitativen Eigenschaften der Wirkstoffformulierung zu verbessern. Bevorzugt entfalten diese Stoffe keine oder im Kontext mit der angestrebten Therapie keine nennenswerte oder zumindest keine unerwünschte pharmakologische Wirkung. Zu den Hilfs- und Zusatzstoffen zählen z.B. weitere Stabilisatoren, Komplexbildner, Antioxidantien und/oder Konservierungsstoffe, die die Verwendungsdauer der fertigen Arzneimittelformulierung verlängern, Geschmackstoffe, Vitamine und/oder sonstige dem Stand der Technik bekannte Zusatzstoffe. Zu den Zusatzstoffen zählen auch pharmakologisch unbedenkliche Salze wie beispielsweise Natriumchlorid.

Zu den bevorzugten Hilfsstoffen zählen Antioxidantien, wie beispielsweise Ascorbinsäure, sofern nicht bereits für die Einstellung des pH-Werts verwendet, Vitamin A, Vitamin E, Tocopherole und ähnliche im menschlichen Organismus vorkommende Vitamine oder Provitamine.

Konservierungsstoffe können eingesetzt werden, um die Formulierung vor Kontamination mit pathogenen Keimen zu schützen. Als Konservierungsstoffe eignen sich die dem Stand der Technik bekannten, insbesondere Benzalkoniumchlorid oder Benzoesäure bzw. Benzoate wie Natriumbenzoat in der aus dem Stand der Technik bekannten Konzentration. Bevorzugt wird der erfindungsgemäßen Formulierung Benzalkoniumchlorid beigemischt. Die Menge des Benzalkoniumchlorids beträgt dabei zwischen 5 mg / 100 ml Formulierung und 20 mg / 100 ml Formulierung, bevorzugt zwischen 5 mg / 100 ml Formulierung und 15 mg / 100 ml Formulierung, stärker bevorzugt zwischen 8 mg / 100 ml Formulierung und 12 mg / 100 ml Formulierung, am stärksten bevorzugt bei 10 mg / 100 ml Formulierung.

Bevorzugte Formulierungen enthalten außer dem Lösungsmittel Wasser und dem Tiotropiumbromid nur noch Benzalkoniumchlorid, Natriumedetat und die zum Einstellen des pH-Werts notwendige Säure, bevorzugt Salzsäure.

Wie bereits erwähnt, wird Tiotropiumbromid in der EP 418 716 A1 beschrieben.

Kristallines Tiotropiumbromid-Monohydrat kann mittels eines Herstellverfahrens, welches nachfolgend detaillierter beschrieben wird, erhalten werden.

Zur Herstellung des kristallinen Monohydrats gemäß der vorliegenden Erfindung ist es erforderlich, Tiotropiumbromid, welches beispielsweise nach der in der EP 418 716 A1 offenbarten Herstellungsvorschrift erhalten worden ist, in Wasser aufzunehmen, zu Erwärmen, eine Reinigung mit Aktivkohle durchzuführen und nach Abtrennen der Aktivkohle unter langsamem Abkühlen das Tiotropiumbromid-Monohydrat langsam zu kristallisieren.

Bevorzugt wird wie nachfolgend beschrieben vorgegangen.

In einem geeignet dimensionierten Reaktionsgefäß wird das Lösemittel mit Tiotropiumbromid, welches beispielsweise nach der in der EP 418 716 A1 offenbarten Herstellungsvorschrift erhalten worden ist, gemischt.

Pro Mol eingesetztes Tiotropiumbromid werden 0,4 bis 1,5 kg, bevorzugt 0,6 bis 1 kg, besonders bevorzugt ca. 0,8 kg Wasser als Lösemittel verwendet.

Die erhaltene Mischung wird unter Rühren erwärmt, vorzugsweise auf mehr als 50°C, besonders bevorzugt auf mehr als 60°C. Die maximal wählbare Temperatur bestimmt sich durch den Siedepunkt des verwendeten Lösemittels Wasser. Vorzugsweise wird die Mischung auf einen Bereich von 80-90°C erhitzt.

In diese Lösung wird Aktivkohle, trocken oder wasserfeucht, eingebracht. Bevorzugt werden pro Mol eingesetztes Tiotropiumbromid 10 bis 50 g, besonders bevorzugt 15 bis 35 g, höchst bevorzugt etwa 25 g Aktivkohle eingesetzt. Gegebenenfalls wird die Aktivkohle vor Einbringen in die Tiotropiumbromid-haltige Lösung in Wasser aufgeschlämmt. Pro Mol eingesetztes Tiotropiumbromid werden zum Aufschlämmen der Aktivkohle 70 bis 200 g, bevorzugt 100 bis 160 g, besonders bevorzugt ca. 135 g Wasser verwendet. Wird die Aktivkohle vor Einbringen in die Tiotropiumbromid-haltige Lösung zuvor in Wasser aufgeschlämmt, empfiehlt es sich, mit der gleichen Menge Wasser nachzuspülen.

Bei konstanter Temperatur wird nach erfolgter Aktivkohlezugabe zwischen 5 bis 60 Minuten, bevorzugt zwischen 10 und 30 Minuten, besonders bevorzugt etwa 15 Minuten weitergerührt und die erhaltene Mischung filtriert, um die Aktivkohle zu entfernen. Der Filter wird anschließend mit Wasser nachgespült. Hierfür werden pro Mol eingesetztes Tiotropiumbromid 140 bis 400 g, bevorzugt 200 bis 320 g, höchst bevorzugt ca. 270 g Wasser verwendet.

Das Filtrat wird anschließend langsam abgekühlt, vorzugsweise auf eine Temperatur von 20-25°C. Die Abkühlung wird vorzugsweise mit einer Abkühlrate von 1 bis 10°C pro 10 bis 30 Minuten, bevorzugt von 2 bis 8°C pro 10 bis 30 Minuten, besonders bevorzugt von 3 bis 5°C pro 10 bis 20 Minuten, höchst bevorzugt von 3 bis 5°C pro ca. 20 Minuten durchgeführt. Gegebenenfalls kann sich nach dem Abkühlen auf 20 bis 25°C eine weitere Abkühlung auf unter 20°C, besonders bevorzugt auf 10 bis 15°C anschließen.

Nach erfolgter Abkühlung wird zwischen 20 Minuten und 3 Stunden, vorzugsweise zwischen 40 Minuten und 2 Stunden, besonders bevorzugt etwa eine Stunde zur Vervollständigung der Kristallisation nachgerührt.

Die entstandenen Kristalle werden abschließend durch Filtrieren oder Absaugen des Lösemittels isoliert. Sollte es erforderlich sein, die erhaltenen Kristalle einem weiteren Waschschritt zu unterwerfen, empfiehlt es sich als Waschlösemittel Wasser oder Aceton zu verwenden. Pro Mol eingesetztes Tiotropiumbromid können zum Waschen der erhaltenen Tiotropiumbromid-monohydrat-Kristalle 0,1 bis 1,0 L, bevorzugt 0,2 bis 0,5 L, besonders bevorzugt etwa 0,3 L Lösemittel Verwendung finden. Gegebenenfalls kann der Waschschritt wiederholt durchgeführt werden.

Das erhaltene Produkt wird im Vakuum oder mittels erwärmter Umluft bis zum Erreichen eines Wassergehalts von 2,5 - 4,0 % getrocknet.

Ein Aspekt der vorliegenden Erfindung betrifft daher auch Lösungsformulierungen der oben beschriebenen Art, bei denen kristallines Tiotropiumbromid-Monohydrat eingesetzt wird, welches gemäß vorstehend beschriebener Vorgehensweise erhältlich ist.

Die erfindungsgemäßen Arzneimittelformulierungen mit Tiotropiumbromid werden bevorzugt in einem Inhalator der vorstehend beschriebenen Art verwendet, um daraus die erfindungsgemäßen treibgasfreien Aerosole herzustellen. An dieser Stelle sei deshalb noch einmal ausdrücklich auf die eingangs beschriebenen Patentdokumente verwiesen, auf die hiermit Bezug genommen wird.

Wie eingangs geschildert wird eine weiterentwickelte Ausführungsform des bevorzugten Inhalators in der WO 97/12687 und deren Figuren 6 offenbart. Dieser Vernebler (Respimat®) kann vorteilhaft zur Erzeugung der erfindungsgemäßen inhalierbaren Aerosole mit einem Tiotropiumsalz als Wirkstoff eingesetzt werden.

Aufgrund seiner zylinderähnlichen Form und einer handlichen Größe von weniger als 9 bis 15 cm in der Länge und 2 bis 4 cm in der Breite kann dieses Device jederzeit vom Patienten mitgeführt werden. Der Vernebler versprüht ein definiertes Volumen der Arzneimittelformulierung unter Anwendung hoher Drücke durch kleine Düsen, so daß inhalierbare Aerosole entstehen.

Im wesentlichen besteht der bevorzugte Zerstäuber aus einem Gehäuseoberteil, einem Pumpengehäuse, einer Düse, einem Sperrspannwerk, einem Federgehäuse, einer Feder und einem Vorratsbehälter, gekennzeichnet durch
- ein Pumpengehäuse, das im Gehäuseoberteil befestigt ist, und das an seinem einen Ende einen Düsenkörper mit der Düse bzw. Düsenanordnung trägt,
- einen Hohlkolben mit Ventilkörper,
- einen Abtriebsflansch, in dem der Hohlkolben befestigt ist, und der sich im Gehäuseoberteil befindet,
- ein Sperrspannwerk, das sich im Gehäuseoberteil befindet,
- ein Federgehäuse mit der darin befindlichen Feder, das am Gehäuseoberteil mittels eines Drehlagers drehbar gelagert ist,
- ein Gehäuseunterteil, das auf das Federgehäuse in axialer Richtung aufgesteckt ist.

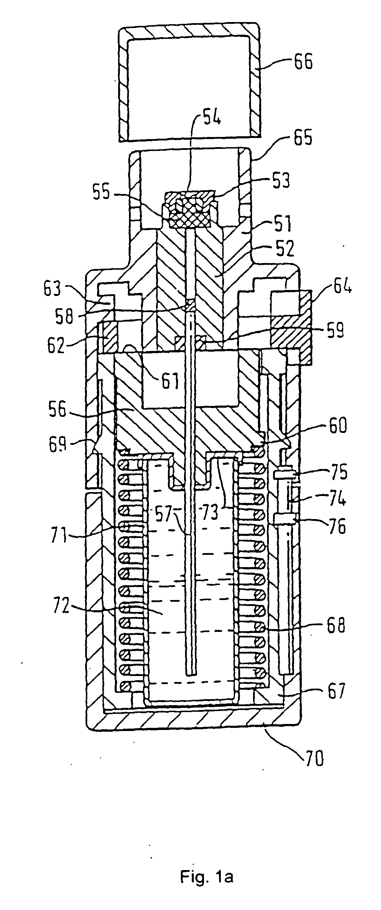

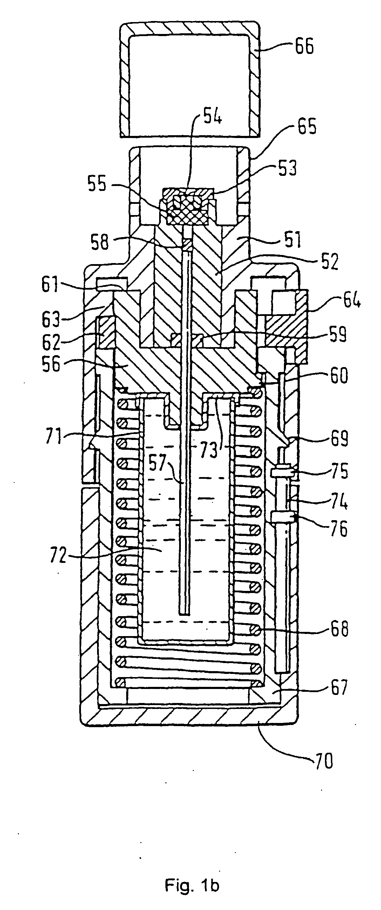

Der Hohlkolben mit Ventilkörper entspricht einer in der WO 97/12687 offenbarten Vorrichtungen. Er ragt teilweise in den Zylinder des Pumpengehäuses hinein und ist im Zylinder axial verschiebbar angeordnet. Insbesondere wird auf die Figuren 1-4 - insbesondere Figur 3 - und die dazugehörigen Beschreibungsteile Bezug genommen. Der Hohlkolben mit Ventilkörper übt auf seiner Hochdruckseite zum Zeitpunkt des Auslösens der Feder einen Druck von 5 bis 60 MPa (etwa 50 bis 600 bar), bevorzugt 10 bis 60 MPa (etwa 100 bis 600 bar) auf das Fluid, die abgemessene Wirkstofflösung aus. Dabei werden Volumina von 10 bis 50 Mikroliter bevorzugt, besonders bevorzugt sind Volumina von 10 bis 20 Mikroliter, ganz besonders bevorzugt ist ein Volumen von 10 bis 15 Mikroliter pro Hub.

Der Ventilkörper ist bevorzugt an dem Ende des Hohlkolbens angebracht, das dem Düsenkörper zugewandt ist.

Die Düse im Düsenkörper ist bevorzugt mikrostrukturiert, d.h. durch Mikrotechnik hergestellt. Mikrostrukturierte Düsenkörper sind beispielsweise in der WO-99/16530 offenbart; auf diese Schrift wird hiermit inhaltlich Bezug genommen, insbesondere auf Figur 1 und deren Beschreibung.

Der Düsenkörper besteht z.B. aus zwei fest miteinander verbundenen Platten aus Glas und/oder Silizium, von denen wenigstens eine Platte einen oder mehrere mikrostrukturierte Kanäle aufweist, die die Düseneinlaßseite mit der Düsenauslaßseite verbinden. Auf der Düsenauslaßseite ist mindestens eine runde oder nicht-runde Öffnung von 2 bis 10 Mikrometer Tiefe und 5 bis 15 Mikrometern Breite, wobei die Tiefe bevorzugt bei 4, 5 bis 6,5 Mikrometern und die Länge bei 7 bis 9 Mikrometern beträgt.

Im Fall von mehreren Düsenöffnungen, bevorzugt sind zwei, können die Strahlrichtungen der Düsen im Düsenkörper parallel zueinander verlaufen oder sie sind in Richtung Düsenöffnung gegeneinander geneigt. Bei einem Düsenkörper mit mindestens zwei Düsenöffnungen auf der Auslaßseite können die Strahlrichtungen mit einem Winkel von 20 Grad bis 160 Grad gegeneinander geneigt sein, bevorzugt wird ein Winkel von 60 bis 150 Grad, insbesondere bevorzugt 80 bis 100°.

Die Düsenöffnungen sind bevorzugt in einer Entfernung von 10 bis 200 Mikrometern angeordnet, stärker bevorzugt in einer Entfernung von 10 bis 100 Mikrometer, besonders bevorzugt 30 bis 70 Mikrometer. Am stärksten bevorzugt sind 50 Mikrometer.

Die Strahlrichtungen treffen sich dementsprechend in der Umgebung der Düsenöffnungen.

Die flüssige Arzneimittelzubereitung trifft wie bereits erwähnt mit einem Eingangsdruck von bis zu 600 bar, bevorzugt 200 bis 300 bar auf den Düsenkörper und wird über die Düsenöffnungen in ein inhalierbares Aerosol zerstäubt. Die bevorzugten Teilchengrößen des Aerosols liegen bei bis zu 20 Mikrometern, bevorzugt 3 bis 10 Mikrometern.

Das Sperrspannwerk enthält eine Feder, bevorzugt eine zylindrische schraubenförmige Druckfeder, als Speicher für die mechanische Energie. Die Feder wirkt auf den Abtriebsflansch als Sprungstück, dessen Bewegung durch die Position eines Sperrglieds bestimmt wird. Der Weg des Abtriebsflansches wird durch einen oberen und einen unteren Anschlag präzise begrenzt. Die Feder wird bevorzugt über ein kraftübersetzendes Getriebe, z.B. ein Schraubschubgetriebe, durch ein äußeres Drehmoment gespannt, das beim Drehen des Gehäuseoberteils gegen das Federgehäuse im Gehäuseunterteil erzeugt wird. In diesem Fall enthalten das Gehäuseoberteil und der Abtriebsflansch ein ein- oder mehrgängiges Keilgetriebe.

Das Sperrglied mit einrückenden Sperrflächen ist ringförmig um den Abtriebsflansch angeordnet. Es besteht z.B. aus einem in sich radial elastisch verformbaren Ring aus Kunststoff oder aus Metall. Der Ring ist in einer Ebene senkrecht zur Zerstäuberachse angeordnet. Nach dem Spannen der Feder schieben sich die Sperrflächen des Sperrgliedes in den Weg des Abtriebsflansches und verhindern das Entspannen der Feder. Das Sprerrglied wird mittels einer Taste ausgelöst. Die Auslösetaste ist mit dem Sperrglied verbunden oder gekoppelt. Zum Auslösen des Sperrspannwerkes wird die Auslösetaste parallel zur Ringebene, und zwar bevorzugt in den Zerstäuber hinein, verschoben; dabei wird der verformbare Ring in der Ringebene verformt. Konstruktive Details des Sperrspannwerkes sind in der WO 97/20590 beschrieben.

Das Gehäuseunterteil wird in axialer Richtung über das Federgehäuse geschoben und verdeckt die Lagerung, den Antrieb der Spindel und den Vorratsbehälter für das Fluid.

Beim Betätigen des Zerstäubers wird das Gehäuseoberteil gegen das Gehäuseunterteil gedreht, wobei das Gehäuseunterteil das Federgehäuse mitnimmt. Dabei wird die Feder über das Schraubschubgetriebe zusammengedrückt und gespannt, und das Sperrwerk rastet selbsttätig ein. Der Drehwinkel ist bevorzugt ein ganzzahliger Bruchteil von 360 Grad, z.B. 180 Grad. Gleichzeitig mit dem Spannen der Feder wird das Abtriebsteil im Gehäuseoberteil um einen vorgegebenen Weg verschoben, der Hohlkolben wird innerhalb des Zylinders im Pumpengehäuse zurückgezogen, wodurch eine Teilmenge des Fluids aus dem Vorratsbehälter in den Hochdruckraum vor der Düse eingesaugt wird.

In den Zerstäuber können gegebenenfalls nacheinander mehrere das zu zerstäubende Fluid enthaltende austauschbare Vorratsbehälter eingeschoben und benutzt werden. Der Vorratsbehälter enthält die erfindungsgemäße wässerige Aerosolzubereitung.

Der Zerstäubungsvorgang wird durch leichtes Eindrücken der Auslösetaste eingeleitet. Dabei gibt das Sperrwerk den Weg für das Abtriebsteil frei. Die gespannte Feder schiebt den Kolben in den Zylinder des Pumpengehäuses hinein. Das Fluid tritt aus der Düse des Zerstäubers in zerstäubter Form aus.

Weitere konstruktive Details sind in den PCT-Anmeldungen WO 97/12683 und WO 97/20590 offenbart.

Die Bauteile des Zerstäubers (Verneblers) sind aus einem der Funktion entsprechend geeignetem Material. Das Gehäuse des Zerstäubers und - so weit es die Funktion erlaubt - auch andere Teile sind bevorzugt aus Kunststoff, z.B. im Spritzgießverfahren, hergestellt. Für medizinische Zwecke werden physiologisch unbedenkliche Materialien verwendet.

In den Figuren 1a/b, die identisch sind mit den Figuren 6 a/b der WO 97/12687, ist der Vernebler (Respimat®) beschrieben, mit dem die erfindungsgemäßen wäßrigen Aerosolzubereitungen vorteilhaft inhaliert werden können.

Figur 1 a zeigt einen Längsschnitt durch den Zerstäuber bei gespannter Feder, Figur 1 b zeigt einen Längsschnitt durch den Zerstäuber bei entspannter Feder.

Das Gehäuseoberteil (51) enthält das Pumpengehäuse (52), an dessen Ende der Halter (53) für die Zerstäuberdüse angebracht ist. In dem Halter befindet sich der Düsenkörper (54) und ein Filter (55). Der im Abtriebsflansch (56) des Sperrspannwerkes befestigte Hohlkolben (57) ragt teilweise in den Zylinder des Pumpengehäuses hinein. An seinem Ende trägt der Hohlkolben den Ventilkörper (58). Der Hohlkolben ist mittels der Dichtung (59) abgedichtet. Innerhalb des Gehäuseoberteils befindet sich der Anschlag (60), an dem der Abtriebsflansch bei entspannter Feder anliegt. Am Abtriebsflansch befindet sich der Anschlag (61), an dem der Abtriebsflansch bei gespannter Feder anliegt. Nach dem Spannen der Feder schiebt sich das Sperrglied (62) zwischen den Anschlag (61) und eine Abstützung (63) im Gehäuseoberteil. Die Auslösetaste (64) steht mit dem Sperrglied in Verbindung. Das Gehäuseoberteil endet im Mundstück (65) und ist mit der aufsteckbaren Schutzkappe (66) verschlossen.

Das Federgehäuse (67) mit Druckfeder (68) ist mittels der Schnappnasen (69) und Drehlager am Gehäuseoberteil drehbar gelagert. Über das Federgehäuse ist das Gehäuseunterteil (70) geschoben. Innerhalb des Federgehäuses befindet sich der austauschbare Vorratsbehälter (71) für das zu zerstäubende Fluid (72). Der Vorratsbehälter ist mit dem Stopfen (73) verschlossen, durch den der Hohlkolben in den Vorratsbehälter hineinragt und mit seinem Ende in das Fluid (Vorrat an Wirkstofflösung) eintaucht.

In der Mantelfläche des Federgehäuses ist die Spindel (74) für das mechanische Zählwerk angebracht. An dem Ende der Spindel, das dem Gehäuseoberteil zugewandt ist, befindet das Antriebsritzel (75). Auf der Spindel sitzt der Reiter (76).

Der oben beschriebene Vernebler ist geeignet, die erfindungsgemäßen Aerosolzubereitungen zu einem für die Inhalation geeignetem Aerosol zu vernebeln.

Wird die erfindungsgemäße Formulierung mittels der vorstehend beschriebenen Technik (Respimat^{®}) vernebelt, sollte die ausgebrachte Masse bei wenigstens 97%, bevorzugt wenigstens 98% aller Betätigungen des Inhalators (Hub oder Hübe) einer definierten Menge mit einem Toleranzbereichs von maximal 25%, bevorzugt 20% dieser Menge entsprechen. Bevorzugt werden pro Hub zwischen 5 und 30 mg Formulierung als definierte Masse ausgebracht, besonders bevorzugt zwischen 5 und 20 mg.

Die erfindungsgemäße Formulierung kann jedoch auch mittels anderer als der vorstehend beschriebenen Inhalatoren, beispielsweise Jet-Stream-Inhalatoren, vernebelt werden.

### Beispiele

### I. Synthesebeispiel für Tiotropiumbromid-Monohydrat

In einem geeigneten Reaktionsgefäß werden in 25,7 kg Wasser 15,0 kg Tiotropiumbromid eingetragen. Die Mischung wird auf 80-90°C erhitzt und bei gleichbleibender Temperatur solange gerührt, bis eine klare Lösung entsteht. Aktivkohle (0,8 kg), wasserfeucht, wird in 4,4 kg Wasser aufgeschlämmt, diese Mischung in die Tiotropiumbromid-haltige Lösung eingetragen und mit 4,3 kg Wasser nachgespült. Die so erhaltene Mischung wird wenigstens 15 Min. bei 80-90°C gerührt und anschließend über einen beheizten Filter in einen auf 70°C Manteltemperatur vorgewärmten Apparat filtriert. Der Filter wird mit 8,6 kg Wasser nachgespült. Der Apparateinhalt wird mit 3-5°C pro 20 Minuten auf eine Temperatur von 20-25°C abgekühlt. Mit Kaltwasserkühlung wird der Apparat auf 10-15°C weiter abgekühlt und die Kristallisation durch mindestens einstündiges Nachrühren vervollständigt. Das Kristallisat wird über einen Nutschentrockner isoliert, der isolierte Kristallbrei mit 9 L kaltem Wasser (10-15°C) und kaltem Aceton (10-15°C) gewaschen. Die erhaltenen Kristalle werden bei 25°C über 2 Stunden im Stickstoffstrom getrocknet. Ausbeute : 13,4 kg Tiotropiumbromid-Monohydrat (86 % d. Th.)

### II. Formulierungsbeispiele

100 ml Arzneimittelzubereitung enthalten:

| Beispiel | Menge an Tiotropiumbromid mit einem Anteil bezogen auf Tiotropium: | Menge an Tiotropiumbromid-Monohydrat | Menge an Benzalkoniumchlorid | Menge an Natriumedetat | pH-Wert, eingestellt mit HCl (1N) |
|---|---|---|---|---|---|
| 1 | 0,045 g | --- | 10 mg | 10 mg | 2,9 |
| 2 | | 0,057 | 10 mg | 10 mg | 2,9 |
| 3 | 0,023 g | --- | 10 mg | 10 mg | 2,9 |
| 4 | | 0,028 | 10 mg | 10 mg | 2,9 |
| 5 | 0,045 g | --- | 10 mg | 10 mg | 2,8 |
| 6 | | 0,057 | 10 mg | 10 mg | 2,8 |
| 7 | 0,023 g | --- | 10 mg | 10 mg | 2,8 |
| 8 | | 0,028 | 10 mg | 10 mg | 2,8 |
| 9 | 0,045 g | --- | 10 mg | 10 mg | 3,0 |
| 10 | | 0,057 | 10 mg | 10 mg | 3,0 |
| 11 | 0,023 g | --- | 10 mg | 10 mg | 3,0 |
| 12 | | 0,028 | 10 mg | 10 mg | 3,0 |

Der restliche Bestandteil ist geeinigtes Wasser bzw. Wasser für Injektionszwecke mit einer Dichte von 1,00 g/cm³ bei einer Temperatur von 15°C bis 31 °C.

Weitere Beispiele 13 bis 24:
Analog Beispiele 1 bis 12, aber mit 9 mg Natriumedetat.

Weitere Beispiele 25 bis 36:
Analog Beispiele 1 bis 12, aber mit 11 mg Natriumedetat.

Weitere Beispiele 37 bis 48:
Analog Beispiele 1 bis 12, aber mit 9 mg Benzalkoniumchlorid.

Weitere Beispiele 49 bis 60:
Analog Beispiele 1 bis 12, aber mit 11 mg Benzalkoniumchlorid.

In weiteren Beispielen beträgt die Menge an Benzalkoniumchlorid 8 bzw. 12 mg.

In weiteren Beispielen beträgt die Menge an Natriumedetat 8 bzw. 12 mg.

Unter den Beispielen sind die Beispiele 1 bis 4 am stärksten bevorzugt.

## Patentansprüche

1. Arzneimittelzubereitung bestehend aus
Tiotropiumbromid als Wirkstoff, in einer Konzentration bezogen auf Tiotropium zwischen 0,02 g pro 100 ml Formulierung und 0,05 g pro 100 ml Formulierung, wobei das Tiotropiumbromid in der Arzneimittelzubereitung vollständig gelöst vorliegt,
- Wasser als alleinigem Lösungsmittel,
- Säure zum Einstellen eines pH-Werts zwischen 2,7 und 3,1 bevorzugt 2,8 und 3,05,
- Benzalkoniumchlorid in einer Konzentration zwischen 8 mg /100ml und 12 mg/100ml,
- Natriumedetat in einer Menge zwischen 8 mg / 100 ml Formulierung und 12 mg / 100 ml Formulierung .

2. Arzneimittelzubereitung nach Anspruch 1, **dadurch gekennzeichnet, daß** der pH-Wert zwischen 2,8 und 3,0, bevorzugt bei 2,9 liegt.

3. Arzneimittelzubereitung nach einem der Ansprüche 1 bis 2, **dadurch gekennzeichnet, daß** der pH-Wert mit einer anorganischen Säure, bevorzugt Salzsäure eingestellt wird.

4. Arzneimittelzubereitung nach einem der Ansprüche 1 bis 3, **dadurch gekennzeichnet, daß** 100 ml der Formulierung hergestellt werden durch Lösen von 0,057 g Tiotropiumbromid-Monohydrat, 10 mg wasserfreiem Benzalkoniumchlorid, 10 mg Natriumedetat in Wasser ad 100 ml und 1 N Salzsäure zum Einstellen eines pH-Werts von 2,9.

5. Arzneimittelzubereitung nach einem der Ansprüche 1 bis 3, **dadurch gekennzeichnet, daß** 100 ml der Formulierung hergestellt werden durch Lösen von 0,028 g Tiotropiumbromid-Monohydrat, 10 mg wasserfreiem Benzalkoniumchlorid, 10 mg Natriumedetat in Wasser ad 100 ml und 1 N Salzsäure zum Einstellen eines pH-Werts von 2,9.

6. Verwendung einer Arzneimittelzubereitung nach einem der Ansprüche 1 bis 5 zur Herstellung eines Arzneimittels zur Behandlung von Asthma und/oder COPD.

## Claims

1. Pharmaceutical preparation consisting of
• tiotropium bromide as active substance, in a concentration based on tiotropium of between 0.02 g per 100 ml of formulation and 0.05 g per 100 ml of formulation, the tiotropium bromide being present in the pharmaceutical preparation in totally dissolved form
• water as the only solvent,
• acid for adjusting the pH to between 2.7 and 3.1, preferably 2.8 and 3.05,
• benzalkonium chloride in a concentration of between 8 mg/100 ml and 12 mg/100 ml,
• sodium edetate in an amount of between 8 mg/100 ml of formulation and 12 mg/100 ml of formulation.

2. Pharmaceutical preparation according to claim 1, **characterised in that** the pH is between 2.8 and 3.0, preferably 2.9.

3. Pharmaceutical preparation according to one of claims 1 to 2, **characterised in that** the pH is adjusted with an inorganic acid, preferably hydrochloric acid.

4. Pharmaceutical preparation according to one of claims 1 to 3, **characterised in that** 100 ml of the formulation are prepared by dissolving 0.057 g of tiotropium bromide monohydrate, 10 mg of anhydrous benzalkonium chloride, 10 mg of sodium edetate in water ad 100 ml and IN hydrochloric acid for adjusting the pH to 2.9.

5. Pharmaceutical preparation according to one of claims 1 to 3, **characterised in that** 100 ml of the formulation are prepared by dissolving 0.028 g of tiotropium bromide monohydrate, 10 mg of anhydrous benzalkonium chloride, 10 mg of sodium edetate in water ad 100 ml and IN hydrochloric acid for adjusting the pH to 2.9.

6. Use of a pharmaceutical preparation according to one of claims 1 to 5 for preparing a medicament for treating asthma and/or COPD.

## Revendications

1. Préparation médicamenteuse consistant en bromure de tiotropium comme principe actif, en une concentration rapportée au tiotropium entre 0,02 g pour 100 ml de formulation et 0,05 g pour 100 ml de formulation, où le bromure de tiotropium est présent dans la préparation médicamenteuse à l'état totalement dissous,
- de l'eau comme seul solvant,
- un acide pour établir un pH entre 2,7 et 3,1, de préférence de 2,8 à 3,05,
- du chlorure de benzalkonium en une concentration entre 8 mg/100 ml et 12 mg/100 ml,
- de l'édétate de sodium en une quantité entre 8 mg/100 ml de formulation et 12 mg/100 ml de formulation.

2. Préparation médicamenteuse selon la revendication 1 **caractérisée en ce que** le pH est situé entre 2,8 et 3,0, de préférence à 2,9.

3. Préparation médicamenteuse selon l'une des revendications 1 à 2 **caractérisée en ce que** le pH est établi avec un acide inorganique, de préférence l'acide chlorhydrique.

4. Préparation médicamenteuse selon l'une des revendications 1 à 3 **caractérisée en ce que** 100 ml de formulation sont produits par dissolution de 0,057 g de monohydrate de bromure de tiotropium, 10 mg de chlorure de benzalkonium anhydre, 10 mg d'édétate de sodium dans l'eau pour 100 ml et de l'acide chlorhydrique 1N pour établir un pH de 2,9.

5. Préparation médicamenteuse selon l'une des revendications 1 à 3 **caractérisée en ce que** 100 ml de formulation sont produits par dissolution de 0,028 g de monohydrate de bromure de tiotropium, 10 mg de chlorure de benzalkonium anhydre, 10 mg d'édétate de sodium dans l'eau pour 100 ml et de l'acide chlorhydrique 1N pour établir un pH de 2,9.

6. Utilisation d'une préparation médicamenteuse selon l'une des revendications 1 à 5 pour la production d'un médicament pour le traitement de l'asthme et/ou de la COPD.
